# EUROPEAN PATENT SPECIFICATION

(11) **EP 1 151 010 B1**
(45) Date of publication and mention of the grant of the patent: **26.10.2005**
(21) Application number: 00907506.0
(22) Date of filing: 04.02.2000
(51) Int. Cl.: C07K 16/08, C07K 16/18, C07K 16/28, C07K 16/30, C07K 16/24, A61K 39/395

(54) **HUMAN POLYCLONAL ANTIBODIES FROM TRANSGENIC NONHUMAN ANIMALS**
AUS NICHT-MENSCHLICHEN TRANSGENEN TIEREN GEWONNENE MENSCHLICHE POLYKLONALE ANTIKÖRPER
ANTICORPS HUMAINS POLYCLONAUX D'ANIMAUX GENETIQUEMENT MODIFIES

(30) Priority: 05.02.1999 US 118810 P; 28.04.1999 US 131398 P; 18.05.1999 US 134674 P
(43) Date of publication of application: 07.11.2001
(60) Divisional application: 05018086.8
(73) Proprietor: Therapeutic Human Polyclonals, Inc., Mountain View, CA 94043 (US)
(72) Inventor: Bülow, Jens-Ulrich, East Fremantle WA 6158 (AU)
(74) Representative: HOFFMANN EITLE
(86) International application number: PCT/EP2000/000933
(87) International publication number: WO 2000/046251

(56) References cited:
- EP-A- 1 288 229
- WO-A-00/26373
- WO-A-00/31141
- WO-A-90/04036
- WO-A-93/12227
- US-A- 5 814 318
- WEIDLE U H ET AL: "Genes encoding a mouse monoclonal antibody are expressed in transgenic mice, rabbits and pigs." GENE, (1991 FEB 15) 98 (2) 185-91. , XP000919055
- FIRST N L: "New animal breeding techniques and their application." JOURNAL OF REPRODUCTION AND FERTILITY. SUPPLEMENT, (1990) 41 3-14. REF: 103 , XP000941129
- NATURE BIOTECHNOLOGY, vol. 20, no. 9, September 2002 (2002-09), pages 889-894,

## Description

### Field of the Invention

The field of this invention is substantially human polyclonal antisera for prophylactic and therapeutic treatment of humans.

### Background

The therapy of infectious diseases caused by bacteria, fungi, virus and parasites is largely based on chemotherapy. However, the emergence of drug-resistant organisms requires the continuous development of new antibiotics. At the same time the control of infections is threatened by the emergence of new pathogens. The increasing number of immunocompromised individuals due to malnutrition, AIDS, medical therapies of cancer, autoimmune diseases and organ transplantation decreases the efficacy of antibiotic therapy and increases the difficulty of controlling infections.

Therapies of patients with malignancies and cancer are also based on chemotherapy. However, many of these therapies are ineffective and the mortality of diseased patients is high. Advances in monoclonal antibody technology have provided little improvement because of the immunogenicity of the monoclonal antibodies and their lack of potency. Anti-idiotypic antibody responses in patients undergoing monoclonal antibody therapy can render the antibody therapy ineffective.

Therapy of steroid resistant rejection of transplanted organs requires the uses of biological reagents (monoclonal or polyclonal antibody preparations) that reverse the ongoing alloimmune response in the transplant recipient. However, immunogenicity of antibody preparations may render such therapy ineffective and prevent rejection reversal. As a consequence, a transplanted organ may be rejected.
Similarly, antibody therapies of autoimmune disease patients are of limited success due to the immunogenicity of antibody preparations. While humanization of antibodies decreases immunogenicity, the effectiveness of such antibodies is limited by anti-idiotypic antibody responses and the lack of potency of monoclonal antibodies. Non-immunogenic, potent reagents for the modulation of immune responses have to be developed.

Polyclonal antibody therapy for the treatment of infectious diseases was introduced at the end of the last century. By the 1930s, serum therapy was used for treatment of bacterial and viral infections including pneumonia, meningitis, scarlet fever, whooping cough, anthrax, botulism, gangrene, tetanus, brucellosis, dysentery, tularemia, diphtheria, measles, poliomyelitis mumps and chickenpox. However, the systemic administration of animal sera caused fevers, chills, and allergic reactions. Serum sickness occurred in 10-50% of treated individuals.

The potential of using antibodies in the treatment of a variety of indications is very high. The ability to specifically bind to a target entity provides diverse opportunities to sequester and destroy the entity. However, as demonstrated above, there have been many impediments to the use of heterologous and humanized antibodies. The limitations of monoclonal antibodies adds the additional impediment of reduced affinity. Thus, there is a pressing need to find alternative modalities which provide protection against infectious disease and malignancies or the immunomodulation of transplant recipients and autoimmune disease patients.

### Relevant Literature

Antibody-based therapies in infectious diseases were recently reviewed by A. Casadevall and M. D. Scharff, Clinical Infectious Diseases 1995; 150-161.

The use of antibodies for the treatment of cancer and malignancies was recently reviewed by C. Botti, A. Marinetti, S. Nerini-Molteni, and L Ferrari, Int J Biol Markers 1997; 12(4):141-147; D.R. Anderson, A. Grillo-Lopez, C. Varns, and K.S. Chambers, Biochem Soc Trans 1997; 25(2):705-708; C. Renner, L. Trumper, and M. Pfreundschuh, Leukemia 1997; 11 Suppl 2:S55-59; B. Bodey, S.E. Siegel, and H.E. Kaiser, Anticancer Res 1996; 16(2):661-674.

The use of polyclonal antibody preparations for the treatment of transplant rejection was recently reviewed by N. Bonnefoy-Berard and J.P. Revillard, J Heart Lung Transplant 1996; 15(5):435-442; C. Colby, C.A. Stoukides, and T.R. Spitzer, Ann Pharmacother 1996; 30(10):1164-1174; M.J. Dugan, T.E. DeFor, M. Steinbuch, and A.H. Filipovich, Ann Hematol 1997; 75(1-2):41-46.

The use of polyclonal antibody therapies for autoimmune diseases has been described by W. Cendrowski, Boll Ist Sieroter Milan 1997; 58(4):339-343; L.K. Kastrukoff, D.R. McLean, and T.A. McPherson, Can J Neurol Sci 1978; 5(2):175-178; J.E. Walker, M.M Hoehn, and N. Kashiwagi, J Neurol Sci 1976; 29(2-4):303-309.

The depletion of fat cells using antibody preparations has been described by L. De Clercq, J. Mourot, C. Genart, V. Davidts, and C. Boone, J Anim Sci 1997; 75(7):1791-1797; J.T. Wright and G.J. Hausman, Obes Res 1995; 3(3):265-272.

The cloning of animals from cells has been described by T. Wakayama, A.C.F. Perry, M. Zuccotti, K.R. Johnson and R. Yanagachi, Nature 1998; 394:369-374; J.B. Cibelli, S.L. Stice, P.J. Golueke, J.J. Kane, J. Jerry, C. Blackwell, A. Ponce de Leon, and J.M. Robl, Science 1998; 280:1256-1258; J.B. Cibelli, S. L. Stice, P. J. Golueke, J.K. Kane, J. Jerry, C. Blackwell, F. Abel de Leon, and J. Robl, Nature Biotechnology 1998; 16:642-646; A. E. Schnieke, A.J. Kind, W.A. Ritchie, K. Mycock, A.R. Scott, M. Ritchie, I. Wilmut, A. Colman A, and K.H. Campbell, Science 1997; 278(5346):2130-2133; K.H. Campbell, J. McWhir, W.A. Ritchie, and I. Wilmut, Nature 1996; 380(6569):64-66.

Production of antibodies from transgenic animals is described in U.S. Patent nos. 5,814,318; 5,545,807; and 5,570,429. Homologous recombination for chimeric mammalian hosts is exemplified in U.S. Patent no. 5,416,260. A method for introducing DNA into an embryo is described in U.S. Patent no. 5,567,607. Maintenance and expansion of embryonic stem cells is described in U.S. Patent no. 5,453,357.

Further, EP A 1288229 as well as WO A 00/26373 refer predominantly to the production of antisera by using a human or humanised immunoglobulin translocus in non-gene-converting animals. The human immunoglobulin loci described in these references do not contain multiple human Ig coding sequences flanked/separated by multiple non-coding animal-derived sequences. Also, the human or humanised immunoglobulin loci described in these references do not support gene conversion and therefore result in the expression of antibodies containing a variable region encoded by a single V gene segment, even in gene-converting animals. WO A 00/31141 relates to humanised antibodies specific for HPV surface antigen pre-S1 which show binding affinity similar to mouse monoclonal antibody and showed reduced immunogenicities since they have less mouse derived amino acid residues.

### SUMMARY OF THE INVENTION

Polyclonal antisera to a specific antigen, and a transgenic nonhuman animal comprising genetically altered light and heavy chain immunoglobulin loci or at least a portion of human light and heavy chain immunoglobulin loci are provided. A method employing stepwise modification of a domestic animal in which the antibody repertoire is diversified predominantly by gene conversion (e.g rabbits, sheep, pigs, cows) is used. The method involves replacement by homologous recombination of endogenous elements of the immunoglobulin loci with the corresponding human counterparts, in particular, replacement of one or several exons encoding constant regions of heavy and light chain and one or several variable region elements including the one proximal to the D region locus. In animals, where antibody diversity is generated predominantly by gene conversion, replacement of the V region most proximal to the D region with a human V region element results in expression of the human V element in the majority of immunoglobulins. This genetic engineering is followed by breeding hosts of the same species and selecting for a host which is capable of responding to immunization with production of substantially human antisera with host glycosylation, the immunoglobulin having at least a functional portion of the human heavy chain. Animals expressing the substantially human protein sequence immunoglobulins are used for the generation of polyclonal antibody preparations by immunization with immunogens of interest, particularly, immunogens which initiate antibody production which has therapeutic activity. After purification of the antisera, such antisera may be used, by itself or in combination, with other reagents for the depletion of infectious reagents, malignant cells, cancers, undesirable target cells or immunomodulation.

### DESCRIPTION OF THE SPECIFIC EMBODIMENTS

Methods are provided for producing substantially human antisera in a heterologous host by immunizing the host with an immunogen. In particular a polyclonal antisera composition of a transgenic nonhuman animal that specifically recognizes an immunogen, wherein said antisera composition is comprised predominantly of immunoglobulin protein molecules comprising at least a portion of a human heavy chain constant region, and variable region amino acid sequences encoded by fragments of more than one variable (V) region gene, wherein (i) said immunoglobulin protein molecules specifically bind to said immunogen, and (ii) wherein said transgenic nonhuman animal generates antibody diversity predominantly by gene conversion is provided. The host is characterized by; being at least substantially incapable of producing endogenous antisera and capable of predominantly producing substantially human polypeptide antisera upon exposure to an immunogenic substance; and retaining its capability of rearranging the immunoglobulin locus and recombining the V, (D_{H}), J and C regions to produce substantially human protein antisera, which include at least one human immunoglobulin constant region and/or at least one human variable (V) region element. Of particular interest are constant regions of the subclasses of C_{α} or C_{γ}, including any of the C_{γ} subclasses 1, 2, 3 and 4.

DNA fragments encoding human constant regions and variable elements are integrated into the genome by homologous recombination and replace the corresponding endogenous elements.

Various animals, particularly domestic animals, which can provide reasonable volumes of antisera may be employed. The animals generally are at least 1 kg, preferably 2 kg, and may be 5 kg or more when adult, although smaller animals can be used as appropriate. Also the gestation period should be less than 12 months, usually being in the range of 1 to 4 months. Illustrative animals include *Lagomorpha*, e.g. rabbit, ovine, bovine, canine, feline, equine, and the like, excluding murine. The animals are those where diversification of the antibody repertoire is accomplished predominantly by gene conversion (i.e. rabbits, pigs, sheep, cattle). In these animals, replacement of the V region element proximal to the D region with a human V region element will result in the expression of the human V region element in the majority of immunoglobulins.. The described genetic engineering approach of the subject invention is substantially easier than other approaches that have been performed with mice. In mice, however, diversification of the antibody repertoire is accomplished predominantly by gene rearrangement.

Host cells, e.g. fibroblasts, keratinocytes, myocytes, hepatocytes, epithelial cells, or other cells which may be grown and expanded in culture and do not have a rearranged genome, are transformed (genetically modified) by the introduction of DNA fragments into the cells, where the fragments become integrated into the host genome. Introduction may be by a variety of methods, including bare DNA, transfection with a viral vector, fusion, biolistics, liposomes, etc. The particular method will be selected in accordance with the purpose of the introduction of the DNA and the efficiency of integration. Functional immunoglobulin light and heavy chain loci are modified by homologous recombination, by replacing at least a portion of the host heavy chain constant region with at least a functional portion of the human heavy chain constant region and if desired, analogously, the host light chain constant region with a human light chain constant region. Of particular interest is also the replacement of the V region most proximal to the D region with a human V region element. In this way, while some portions of the immunoglobulin are host sequences, the antisera is not likely to cause a strong immune response in view of the great variety of variable regions in the antisera. In animals, where antibody diversity is generated predominantly by gene conversion, replacement of the V region most proximal to the D region with a human V region element results in expression of the human V element in the majority of immunoglobulins. For the replacement of constant regions it is of particular interest to include at least about 2 of the 3 domains C_{H1}, C_{H2}, and C_{H3}, of the constant region, particularly including C_{H3}. To the extent such antisera can function in a host, particularly an immunocompromised host, the reduced number of stages to attain hosts which produce such antisera is attractive.

For integration at a predetermined site, constructs are prepared which include, in sequence, the DNA fragment for integration and a first marker gene bordered by homologous sequences of at least about 30nt and a second marker gene, whereby homologous integration results in loss of the second marker gene. By having the second marker gene providing negative selection--cells with the second marker gene are selected against and removed from the cell mixture; by having the first marker gene providing positive selection--cells having the first marker gene are retained--by using a medium to which the second marker gene is sensitive. In this manner, those cells in which the construct is randomly integrated are decreased. By following with a medium selective for the first marker gene, cells not retaining the first marker gene will be decreased. In this way, the remaining cells should be those having homologous recombination. Desirably, the cells are in a rapidly proliferating status, rather than a non-proliferating status. By employing a growth medium, such as RPMI1640 or DMEM, supplemented with FCS and growth factors, a growth cycle can be induced.

After the cells have been transformed or transfected, the cells are put in a selective medium in accordance with the marker employed, usually an antibiotic resistance or the tk gene. The cells are expanded in culture and then cloned. Individual cells in clones may then be screened for the desired genetic modification. Conveniently, PCR may be used to identify that the desired modification, deletion or integration, has taken place.

The genetic modifications may be a single modification or, if desired, after expansion of cells having the first modification, the cells may then be subjected to a second modification. For example, after replacing the heavy chain constant regions, one could replace the light chain constant regions.

Where an individual modification occurs, one can use a single marker for positive selection and use the same marker repetitively. Where two or more modifications to the same cell are generated, different positive selection markers should be used, in order to independently select at each stage. As already indicated, there are numerous antibiotic resistance genes, which genes may be used in combination, allowing for selection at each stage. Genes useful for selection include neo, tet, cam, tk, pen, mtx, etc. After the host cells have been modified and demonstrated to have the desired modification, the cells may then be fused with enucleated nuclear transfer unit cells, e.g. oocytes or embryonic stem cells, cells which are totipotent and capable of forming a functional neonate. Fusion is performed in accordance with conventional techniques which are well established. See, for example, Cibelli et al., Science (1998) 280:1256. Alternatively, enucleation of oocytes and nuclear transfer can be performed by microsurgery using injection pipettes. (See, for example, Wakayama et al., Nature (1998) 394:369) The resulting functional egg cells are then cultivated in an appropriate medium and transferred into synchronized recipients.
Another method for producing nuclear transfer unit cells is to introduce DNA constructs comprising human transgenes into fertilized eggs. The eggs may then be expanded to provide embyronic stem cells, which are screened for the desired genetic modification and subsequent embryo transfer into foster mothers, where the eggs are brought to term, and the resulting neonates screened for the modified genotype.

The resulting mutated hosts may then be used for breeding with other mutated hosts. For example, hosts having an altered heavy chain immunoglobulin locus may be bred with hosts having an altered light chain immunoglobulin locus to breed a host capable of producing substantially human polypeptide immunoglobulins. The hemizygous siblings containing the two mutated genes are then bred to produce homozygous siblings. Homozygosity may be readily determined by the absence of the undesired gene sequences. After each breeding, the host is assayed for the presence of the genetic modification in its cells, particularly the germ cells, and may be bred to a further generation, usually not more than three generations, to ensure that the modification is stably maintained through successive generations. The genomes of the various offspring may be analyzed for the maintenance of the genetic modifications or, as appropriate, the offspring may be analyzed for the biological change which the genetic modification generated.

Once the host has been generated, the host may now be used to produce antisera under a variety of conditions. Depending upon the use of the antisera, antigens, immunogens comprising a hapten covalently bonded to an antigen, organisms, e.g. viruses and unicellular organisms, alive, attenuated or dead, fragments of organisms, organelles, cells, particularly human cells or fragments of cells, or the like may be used. Thus the antisera may be directed to an antigen, a small organic molecule or a cell, where the various entities may be endogenous or exogenous to the human host. The immunization composition may be administered in any convenient manner, with or without an adjuvant, and may be administered in accordance with a predetermined schedule. The affinity for the immunization composition may then be monitored and the antisera collected when the antisera has the desired specificity and affinity. The affinity of the antisera generally will be at least about 10⁻⁷, usually at least about 10⁻⁸, preferably at least about 10⁻⁹, or higher.

For some application, one may use hosts in which the V element proximal to the D regions has been replaced with various human V region elements. In this way, different immune responses to the same immunogen will be obtained from the different hosts, where the variable region sequence may be as a result of gene conversion, providing different alleles. The antisera from the different hosts may be mixed to provide a broader repertoiree of antibodies. Up to 10 or more different hosts may be employed, depending on the antigen of interest.

Antibody preparations are obtained by fractionating blood of genetically engineered animals expressing human sequence immunoglobulins. A concentrated immunoglobulin fraction may be prepared by chromatography (affinity, ionic exchange, gel filtration, etc.), selective precipitation with salts such as ammonium sulfate, organic solvents such as ethanol, or polymers such as polyethyleneglycol.

The fractionated antibodies may be dissolved or diluted in non-toxic, non-pyrogenic media suitable for intravenous administration in humans, for instance, sterile buffered saline. In some applications, antibody preparations may be applied directly onto epithelium. For such applications, fractionated antibodies may be dissolved in a water soluble gel such as KY-jelly and the like.

The antibody preparations used for administration are generally characterized by containing a polyclonal antibody population, having immunoglobulin concentrations from 0.1 to 100 mg/ml, more usually from 1 to 10 mg/ml. The antibody preparation may contain immunoglobulins of various isotypes. Alternatively, the antibody preparation may contain antibodies of only one isotype, or a number of selected isotypes.

In most instances the antibody preparation will consist of unmodified immunoglobulins. Alternatively, the immunoglobulin fraction may be subject to treatment such as enzymatic digestion (e.g. with pepsin, papain, plasmin, glycosidases, nucleases, etc.), heating, etc, and/or further fractionated.

The antibody preparations generally are administered into the vascular system, conveniently intravenously by injection or infusion via a catheter implanted into an appropriate vein. The antibody preparation is administered at an appropriate rate, generally ranging from about 10 minutes to about 24 hours, more commonly from about 30 minutes to about 6 hours, in accordance with the rate at which the liquid can be accepted by the patient. Administration of the effective dosage may occur in a single infusion or in a series of infusions. Repeated infusions may be administered once a day, once a week once a month, or once every three months, depending on the half-life of the antibody preparation and the clinical indication. For applications on epithelial surfaces the antibody preparations are applied to the surface in need of treatment in an amount sufficient to provide the intended end result, and can be repeated as needed.

The antibody preparations find use in their ability to bind and neutralize antigenic entities in human body tissues that cause disease or that elicit undesired or abnormal immune responses. An "antigenic entity" is herein defined to encompass any soluble or cell-surface bound molecules including proteins, as well as cells or infectious disease-causing organisms or agents that are at least capable of binding to an antibody and preferably also are capable of stimulating an immune response.

Administration of an antibody preparation against an infectious agent as monotherapy or in combination with chemotherapy results in elimination of infectious particles. A single administration of antibodies decreases the number of infectious particles generally 10 to 100 fold, more commonly more than 1000-fold. Similarly, antibody therapy in patients with malignant disease as monotherapy or in combination with chemotherapy reduces the number of malignant cells generally 10 to 100 fold, or more than 1000-fold. Therapy may be repeated over an extended amount of time to assure the complete elimination of infectious particles, malignant cells, etc. In some instances, therapy with antibody preparations will be continued for extended amounts of time in the absence of detectable amounts of infectious particles or undesirable cells. Similarly, the use of antibody therapy for the modulation of immune responses may consist of single or multiple administrations of therapeutic antibodies. Therapy may be continued for extended amounts of time in the absence of any disease symptoms.

The subject treatment may be employed in conjunction with chemotherapy at dosages sufficient to inhibit infectious disease or malignancies. In autoimmune disease patients or transplant recipients antibody therapy may be employed in conjunction with immunosuppressive therapy at dosages sufficient to inhibit immune reactions.

The following examples are offered by way of illustration and not by way of limitation.

### EXPERIMENTAL

### Generation of transgenic rabbits expressing substantially human immunoglobulin

Exons encoding human constant region elements and variable region elements are integrated into the genome of rabbit fibroblasts by homologous recombination. Rabbit fibroblasts are transfected with various linearized DNA constructs containing human immunoglobulin locus elements. Successfully transfected cells are selected and used for the cloning of rabbits.

### Cloning of rabbits

Mature Dutch Belton rabbits are superovulated by subcutaneous injection of follicle stimulating hormone (FSH) every 12 hours (0.3 mg x 2 and 0.4 mg x 4). Ovulation is induced by intravenous administration of 0.5 mg luteinizing hormone (LH) 12 hours after the last FSH injection. Oocytes are recovered by ovidual flush 17 hours after LH injection. Oocytes are mechanically enucleated 16-19 hours after maturation. Chromosome removal is assessed with bisBENZIMIDE (HOECHST 33342, Sigma, St. Louis, MO) dye under ultraviolet light.

Enucleated oocytes are fused with actively dividing fibroblasts by using one electrical pulse of 180 V/cm for 15 us (Electrocell Manipulator 200, Genetronics, San Diego, CA). After 3-5 hours oocytes are chemically activated with calcium ionophore (6 uM) for 4 min (# 407952, Calbiochem, San Diego, CA) and 2 mM 6-dimethylaminopurine (DMAP, Sigma) in CR2 medium (Specialty Media, Lavalett, NJ) with 3 mg/ml bovine serum albumin (fatty acid free, Sigma) for 3 hours. Following the activation, the embryos are washed in hamster embryo culture medium (HECM)-Hepes five times and subsequently, cultivated in CR2 medium containing 3 mg/mgl fatty-acid free BSA for 7 days at 37.8° C and 5%CO2 in air. Embryos are then transferred into synchronized recipients. Offsprings are analyzed by PCR for a segment of the transgene.

### Binding of human antibodies expressed in rabbits to Hepatitis B surface antigen

Genetically engineered rabbits (as described above) are immunized intramuscularly with purified Hepatitis B surface antigen (HBsAg) (10µg in incomplete Freund's adjuvant) on day 0 and day 14. On day 28 animals are bled from the ear and serum is prepared. ELISA plates (NUNC, Denmark) are coated with 1 µg/ml HBsAg in PBS for 1 hour at room temperature. Subsequently, available binding sites are blocked by incubation with 1% non-fat dry milk (NFM) in PBS (300 µl/well). Rabbit serum is diluted in PBS/1%NFM and added to the coated wells. After an incubation of 1 hour, the plates are washed 3 times with PBS/0.05% Tween 20 and bound Ig is detected using goat anti-human Ig conjugated with horse-radish peroxidase. Conjugated goat antibody is detected using o-phenylenediamine dihydrochloride (Sigma) at 1 mg/ml. The colorimetric reaction is stopped by addition of 1 M HCl solution and the absorbance is measured at 490 nm. As a control serum from non-immunized rabbits is used. Serum from non-immunized rabbits does not react with HBsAg. At a dilution of 1:100 the optical density measured in uncoated and HBsAg coated wells is below 0.4. In contrast, serum from immunized rabbits contains substantially human antibodies reactive with HBsAg. At a serum dilution of 1:100 the measured optical density is 2.8. Upon further dilution of the serum the measured optical density declines to 0.2 (at a dilution of 25600). No antibodies reactive with a goat anti-rabbit IgG-HRP conjugate can be detected. This demonstrates that the genetically engineered rabbits produce substantially human anti-HBsAg antibodies following immunization.

### Complement mediated cytotoxicity of virus infection cell line using human antibodies

A human liver carcinoma cell line expressing HBsAg is labeled with 0.1 mCi ⁵¹Cr in 100 ul PBS for 1 hr at 37°C. Two thousand ⁵¹Cr-lableled cells are incubated with serum from genetically engineered rabbits expressing anti-HBsAg immunoglobulin (see above). After two hours at 37°C the release of ⁵¹Cr into the supernatant is determined by measuring radioactivity using a scintillation counter. For the determination of maximum release, 1% Triton X100 is added. The degree of cell lysis is calculated as follows: %Lysis = CPM experimental ±CPM#spontaneous / CPM# total ± CPM spontaneous. Incubation of labeled cells with serum (diluted 1:30) from non-immunized rabbits does not result in cell lysis (<10%). However, incubation of cells with serum from immunized rabbits causes 80% cell lysis. Inactivation of complement in the serum by heat treatment (56°C for 30 minutes) renders the serum from immunized rabbits inactive. These results demonstrate that substantially human antibodies produced by genetically engineered rabbits bind to HBsAg-positive cells and cause complement dependent lysis.

### Treatment of animal with infection.

Substantially human immunoglobulin is purified from the serum of genetically engineered rabbits by ammonium sulfate precipitation and ion exchange chromatography. SCID-mice are injected with one million human liver carcinoma cells expressing HBsAg. Subsequently, 25 µg immunoglobulin is injected peritoneally once per day. Animals treated with antibodies isolated from non-immunized-rabbit serum die after about 60 days. This is similar to untreated recipients of liver carcinoma cells. In contrast, mice treated with antibodies isolated from immunized rabbit serum survive for more than 150 days. This demonstrates that human antibodies produced in genetically engineered rabbits are capable of eliminating human carcinoma cells from SCID-mice.

It is evident from the above results that by using genetically engineered rabbits expressing substantially human immunoglobulin genes, polyclonal antibody preparations against antigens, infectious particles, cancer cells, and the like can be generated. Such polyclonal.antibody preparations can be used to treat patients suffering from an infectious disease or a malignancy. The antisera also can be used to modulate an immune response by elimination of cell subpopulations, cytokines, or the like. The human antibody preparation has a substantially reduced likelihood of engendering an immune response in human patients, as compared to heterologous antisera, it will have few side effects and it can be used safely with positive results.

It will be apparent to one of ordinary skill in the art that many changes and modifications can be made thereto without departing from the scope of the appended claims.

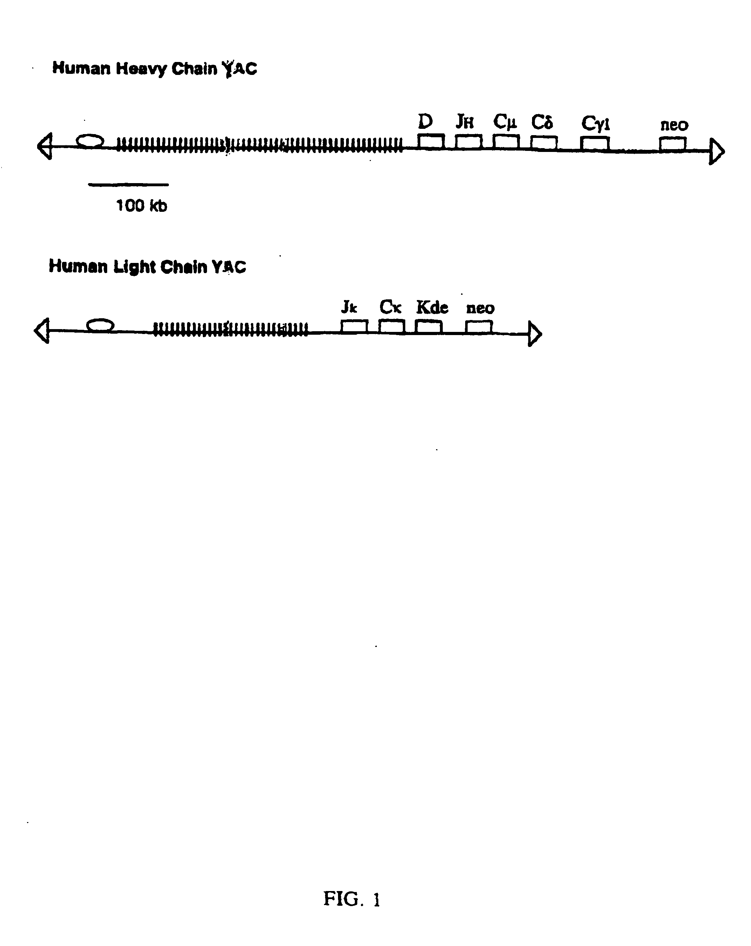

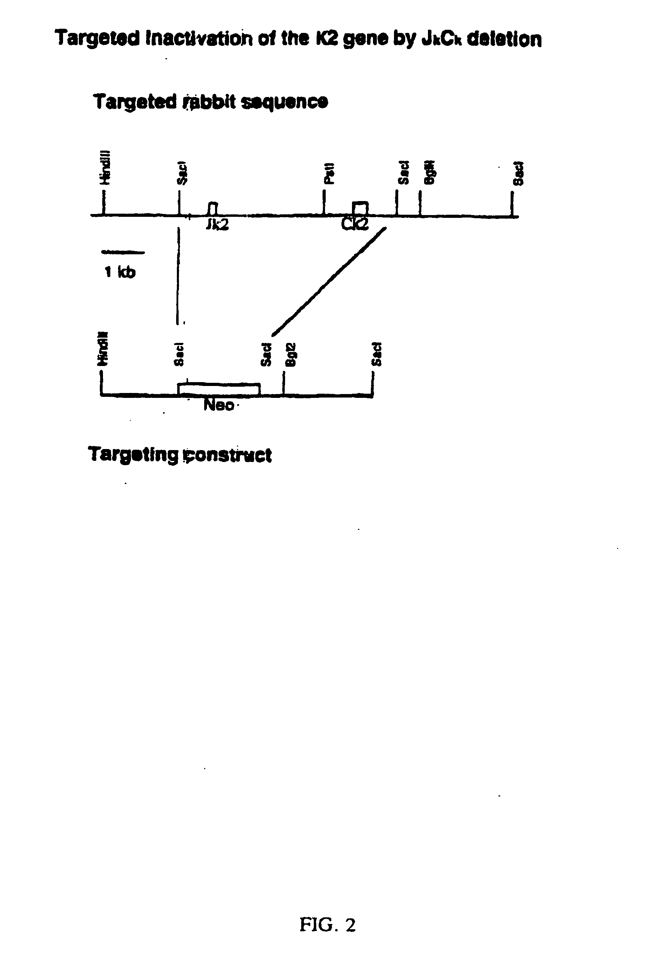

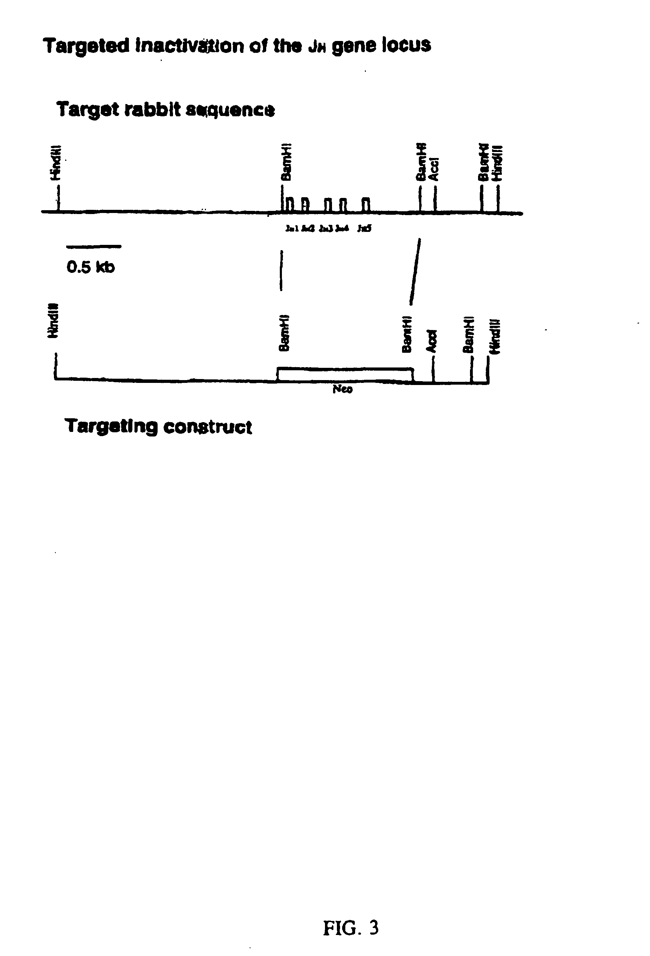

## Claims

1. A polyclonal antisera composition of a transgenic nonhuman animal that specifically recognizes an immunogen, wherein said antisera composition is comprised predominantly of immunoglobulin protein molecules comprising at least a portion of a human heavy chain constant region, and variable region amino acid sequences encoded by fragments of more than one variable (V) region gene, wherein (i) said immunoglobulin protein molecules specifically bind to said immunogen, and (ii) wherein said transgenic nonhuman animal generates antibody diversity predominantly by gene conversion.

2. The polyclonal antisera composition according to Claim 1, wherein said transgenic nonhuman animal comprises at least a portion of functional human heavy chain immunoglobulin genes including more than one V region gene integrated by homologous recombination into its genome.

3. The polyclonal antisera according to Claim 1 or Claim 2 comprised predominantly of immunoglobulin protein molecules comprising at least two of the human heavy chain constant regions C_{H1}, C_{H2}, and C_{H3}.

4. The polyclonal antisera according to Claim 3 comprised predominantly of immunoglobulin protein molecules comprising the human heavy chain constant region C_{H3}.

5. The polyclonal antisera composition according to any one of Claims 1 to 4, wherein in the genome of said nonhuman animal, the V region gene proximal to the D region is replaced with a human V region gene.

6. The polyclonal antisera composition according to Claim 5 comprising antisera from various nonhuman animals in which the V region gene proximal to the D region is replaced with different human V region genes.

7. The polyclonal antisera composition according to Claim 6 comprising antisera from up to 10 different nonhuman animals.

8. The polyclonal antisera composition according to any one of Claims 1 to 7 having an affinity of at least about 10⁻⁷.

9. The polyclonal antisera composition according to any one of Claims 1 to 7 having an affinity of at least about 10⁻⁸.

10. The polyclonal antisera composition according to any one of Claims 1 to 7 having an affinity of at least about 10⁻⁹.

11. The polyclonal antisera composition according to any one of Claims 1 to 10 wherein said transgenic nonhuman animal is from the order of *Lagomorpha*.

12. The polyclonal antisera composition according to Claim 11 wherein said transgenic nonhuman animal is selected from the group consisting of rabbit, ovine, bovine, canine, feline, and equine animals.

13. The polyclonal antisera composition according to Claim 12 wherein said transgenic nonhuman animal is a rabbit, a pig, a sheep, or a cattle.

14. A transgenic nonhuman animal weighing at least 1 kg, comprising at least a portion of functional human heavy chain immunoglobulin genes integrated by homologous recombination into its genome, wherein said portion of functional human heavy chain immunoglobulin genes rearranges in frame with heavy chain immunoglobulin genes endogenous to said nonhuman animal to encode functional antibody molecules that comprise at least a portion of a human heavy chain constant region, and variable region amino acid sequences encoded by fragments of more than one variable (V)region gene, wherein said transgenic nonhuman animal generates antibody diversity predominantly by gene conversion and produces said functional antibody molecules when immunized.

15. A transgenic nonhuman animal weighing at least 1 kg, comprising at least a portion of functional human light chain immunoglobulin genes integrated by homologous recombination into its genome, wherein said portion of functional human light chain immunoglobulin genes rearranges in frame with light chain immunoglobulin genes endogenous to said nonhuman animal to encode functional antibody molecules that comprise at least a portion of a human light chain constant region, and variable region amino acid sequences encoded by fragments of more than one variable (V)region gene, wherein said transgenic nonhuman animal generates antibody diversity predominantly by gene conversion and produces said functional antibody molecules when immunized.

16. The transgenic nonhuman animal according to Claim 14 or Claim 15, wherein said transgenic nonhuman animal is from the order *Lagomorpha*.

17. The transgenic nonhuman animal according to Claim 14, wherein said portion of functional human heavy chain immunoglobulin genes comprises at least one constant region gene.

18. The transgenic nonhuman animal according to Claim 14, wherein said variable region genes comprise the variable region gene proximal to the D region.

19. The transgenic nonhuman animal according to Claim 15 wherein said human immunoglobulin light chain gene encodes the immunoglobulin κ chain.

20. An *in vitro* method for neutralizing an antigenic entity in a human body component, said method comprising:
contacting said body component with an antisera composition according to any of Claims 1-13, wherein the immunoglobulin protein molecules in the antisera composition specifically bind and neutralize said antigenic entity.

21. The method according to Claim 20, wherein said antigenic entity is from an organism that causes an infectious disease.

22. The method according to Claim 20, wherein said antigenic entity is a cell surface molecule.

23. The method according to Claim 22, wherein said cell surface molecule is from a lymphocyte or an adipocyte.

24. The method according to Claim 20, wherein said antigenic entity is a human cytokine or a human chemokine.

25. The method according to Claim 20, wherein said antigenic entity is a cell surface molecule on a malignant cancer cell.

26. The use of an antisera composition according to any of Claims 1-13 for the preparation of a medicament for neutralizing an antigenic entity in a human body component.

27. The use according to Claim 26 wherein said antigenic entity is from an organism that causes an infectious disease.

28. The use according to Claim 26, wherein said antigenic entity is a cell surface molecule.

29. The use according to Claim 28, wherein said cell surface molecule is from a lymphocyte or an adipocyte.

30. The use according to Claim 26, wherein said antigenic entity is a human cytokine or a human chemokine.

31. The use according to Claim 26, wherein said antigenic entity is a cell surface molecule on a malignant cancer cell.

32. A method of producing a transgenic nonhuman animal comprising at least a portion of functional human immunoglobulin genes integrated by homologous recombination into its genome, wherein said portion of functional human immunoglobulin genes rearranges in frame with immunoglobulin genes endogenous to said nonhuman animal to encode functional antibody molecules that comprise at least a portion of human immunoglobulin constant regions, and variable region amino acid sequences encoded by fragments of more than one variable (V) region gene, wherein said transgenic nonhuman animal generates antibody diversity predominantly by gene conversion and produces said functional antibody molecules when immunized, said method comprising:
producing a first mutated animal comprising heavy chain immunoglobulin loci wherein constant and/or variable region genes are replaced with one or several exons of the human heavy chain immunoglobulin locus by genetic alteration of a cell nucleus of said animal, introducing said cell nucleus into a first enucleated nuclear transfer unit cell to provide a first embryonic stem cell, introducing said first nuclear transfer unit cell into a female recipient host to produce a first mutated neonate;
producing a second mutated animal comprising light chain immunoglobulin loci where constant and/or variable region genes are replaced with one or several exons of the human light chain immunoglobulin locus by genetic alteration of a cell nucleus of said animal, introducing said cell nucleus into a second enucleated nuclear transfer unit cell to provide a second embryonic stem cell, introducing said second nuclear transfer unit cell into a female recipient host to produce a second mutated neonate; and
breeding mature first and second mutated neonates and selecting animals capable of producing antisera comprised predominantly of immunoglobulin protein molecules comprising at least a portion of human immunoglobulin constant regions, and variable region amino acid sequences encoded by fragments of more than one variable (V) region gene, wherein (i) said immunoglobulin protein molecules specifically bind to said immunogen, (ii) wherein said transgenic nonhuman animal generates antibody diversity predominantly by gene conversion.

33. A method of producing a transgenic nonhuman animal weighing at least 1 kg, comprising at least a portion of functional human immunoglobulin genes integrated by homologous recombination into its genome, wherein said portion of functional human immunoglobulin genes rearranges in frame with immunoglobulin genes endogenous to said nonhuman animal to encode functional antibody molecules that comprise variable region amino acid sequences encoded by fragments of more than one variable (V) region gene, wherein said transgenic nonhuman animal generates antibody diversity predominantly by gene conversion and produces said functional antibody molecules when immunized, said method comprising:
producing a mutated animal comprising heavy and light chain immunoglobulin loci where constant and/or variable region genes are replaced with one or several exons of the human heavy and/or light chain immunologlobulin locus by genetic alteration of a cell nucleus of said animal, introducing said cell nucleus into an enucleated nuclear transfer unit cell to provide en embryonic stem cell, introducing said nuclear transfer unit cell into a female recipient host to produce mutated neonate; and
breeding mature mutated neonates and selecting animals capable of producing antisera comprised predominantly of immunoglobulin protein molecules comprising at least a portion of a human heavy chain constant region, and variable region amino acid sequences encoded by fragments of more than one variable (V) region gene, wherein (i) said immunoglobulin protein molecules specifically bind to said immunogen, and (ii) wherein said transgenic nonhuman animal generates antibody diversity predominantly by gene conversion.

34. The method according to Claim 33 or Claim 34, wherein said animal is from the order of *Lagomorpha.*

35. The method according to Claim 33 or Claim 34 wherein said animal comprises a variable region gene proximal to the D region.

## Patentansprüche

1. Polyklonale Antiserenzusammensetzung eines transgenen nichtmenschlichen Tiers, die spezifisch ein Immunogen erkennt, wobei die Antiserenzusammensetzung im wesentlichen aus Immunglobulinproteinmolekülen besteht, umfassend mindestens einen Teil einer menschlichen schweren Ketten konstanten Region und variablen Region Aminosäuresequenzen, codiert durch Fragmente von mehr als einem variablen (V)-Regiongen, worin (i) die Immunglobulinproteinmoleküle spezifisch an das Immunogen binden und (ii) wobei das transgene nichtmenschliche Tier eine Antikörperdiversität im wesentlichen durch Genkonversion erzeugt.

2. Polyklonale Antiserenzusammensetzung gemäß Anspruch 1, wobei das transgene nichtmenschliche Tier mindestens einen Teil der funktionalen menschlichen schweren Ketten Immunglobulingene umfaßt, beinhaltend mehr als ein V-Regiongen, integriert durch homologe Rekombination in das Genom.

3. Polyklonale Antiseren gemäß Anspruch 1 oder 2, bestehend im wesentlichen aus Immunglobulinproteinmolekülen, umfassend mindestens zwei der menschlichen schweren Ketten konstanten Regionen C_{H1}, C_{H2} und C_{H3}.

4. Polyklonale Antiseren gemäß Anspruch 3, bestehend im wesentlichen aus Immunglobulinproteinmolekülen, umfassend die menschliche schwere Ketten konstante Region C_{H3}.

5. Polyklonale Antiserenzusammensetzung gemäß einem der Ansprüche 1 bis 4, wobei in dem Genom des nichtmenschlichen Tiers das V-Regiongen proximal zur D-Region durch ein menschliches V-Regiongen ersetzt ist.

6. Polyklonale Antiserenzusammensetzung gemäß Anspruch 5, umfassend Antiseren von verschiedenen nichtmenschlichen Tieren, worin das V-Regiongen proximal zur D-Region durch unterschiedliche menschliche V-Regiongene ersetzt ist.

7. Polyklonale Antiserenzusammensetzung gemäß.Anspruch 6, umfassend Antiseren von bis zu zehn unterschiedlichen nichtmenschlichen Tieren.

8. Polyklonale Antiserenzusammensetzung gemäß einem der Ansprüche 1 bis 7 mit einer Affinität von mindestens ungefähr 10⁻⁷.

9. Polyklonale Antiserenzusammensetzung gemäß einem der Ansprüche 1 bis 7 mit einer Affinität von mindestens ungefähr 10⁻⁸.

10. Polyklonale Antiserenzusammensetzung gemäß einem der Ansprüche 1 bis 7 mit einer Affinität von mindestens ungefähr 10⁻⁹.

11. Polyklonale Antiserenzusammensetzung gemäß einem der Ansprüche 1 bis 10, wobei das transgene nichtmenschliche Tier aus der Ordnung Lagomorpha stammt.

12. Polyklonale Antiserenzusammensetzung gemäß Anspruch 11, wobei das transgene nichtmenschliche Tier ausgewählt ist aus der Gruppe bestehend aus Kaninchen, schweineartigen rinderartigen, hundeartigen, katzenartigen und pferdeartigen Tieren.

13. Polyklonale Antiserenzusammensetzung gemäß Anspruch 12, wobei das transgene nichtmenschliche Tier ein Kaninchen, ein Schwein, ein Schaf oder ein Rind ist.

14. Transgenes nichtmenschliches Tier, das mindestens 1 kg wiegt, umfassend mindestens einen Teil von funktionalen menschlichen schweren Ketten Immunglobulingenen, integriert durch homologe Rekombination ins Genom, wobei der Teil der funktionalen menschlichen schweren Ketten Immunglobulingene sich in frame mit schweren Ketten Immunglobulingenen, die für das nichtmenschliche Tier endogen sind, umanordnet, um funktionelle Antikörpermoleküle zu codieren, die mindestens einen Teil der menschlichen schweren Ketten konstanten Region umfassen und variable Region Aminosäuresequenzen, codiert durch Fragmente von mehr als einem variablen (V)-Regiongen, wobei das transgene nichtmenschliche Tier Antikörperdiversität im wesentlichen durch Genkonversion erzeugt und die funktionellen Antikörpermoleküle erzeugt, wenn es immunisiert wird.

15. Transgenes nichtmenschliches Tier, das mindestens 1 kg wiegt, umfassend mindestens einen Teil von funktionalen menschlichen leichten Ketten Immunglobulingenen, integriert durch homologe Rekombination ins Genom, wobei der Teil der funktionalen in frame mit leichten Ketten Immunglobulingenen, die für das nichtmenschliche Tier endogen sind, umanordnet, um funktionelle Antikörpermoleküle zu codieren, die mindestens einen Teil der menschlichen leichten Ketten konstanten Region umfassen und variable Region Aminosäuresequenzen, codiert durch Fragmente von mehr als einem variablen (V)-Regiongen, wobei das transgene nichtmenschliche Tier Antikörperdiversität im wesentlichen durch Genkonversion erzeugt und die funktionellen Antikörpermoleküle erzeugt, wenn es immunisiert wird.

16. Transgenes nichtmenschliches Tier gemäß Anspruch 14 oder 15, wobei das transgene nichtmenschliche Tier zu der Ordnung Lagomorpha erhört.

17. Transgenes nichtmenschliches Tier gemäß Anspruch 14, wobei der Teil der funktionellen menschlichen schweren Ketten Immunglobulingene mindestens ein konstantes Regiongen umfaßt.

18. Transgenes nichtmenschliches Tier gemäß Anspruch 14, wobei die variablen Regiongene das variable Regiongen proximal zur D-Region umfassen.

19. Transgenes nichtmenschliches Tier gemäß Anspruch 15, wobei das menschliche Immunglobulinleichtkettengen die Immunglobulin-κ-kette codiert.

20. In vitro-Verfahren für die Neutralisierung einer antigenen Einheit in einem menschlichen Körperbestandteil, wobei das Verfahren folgendes umfaßt:
Kontaktieren des Körperbestandteils mit einer Antiserenzusammensetzung gemäß einem der Ansprüche 1 bis 13, wobei die Immunglobulinproteinmoleküle in der Antiserenzusammensetzung spezifisch an die antigene Einheit binden und diese neutralisieren.

21. Verfahren gemäß Anspruch 20, wobei die antigene Einheit von einem Organismus stammt, der eine infektiöse Erkrankung auslöst.

22. Verfahren gemäß Anspruch 20, wobei die antigene Einheit ein Zelloberflächenmolekül ist.

23. Verfahren gemäß Anspruch 22, wobei das Zelloberflächemolekül von einem Lymphozyten oder Adipozyten stammt.

24. Verfahren gemäß Anspruch 20, wobei die antigene Einheit ein menschliches Zytokin oder ein menschlichen Chemokin ist.

25. Verfahren gemäß Anspruch 20, wobei die antigene Einheit ein Zelloberflächenmolekül auf einer malignen Krebszelle ist.

26. Verwendung einer Antiserenzusammensetzung gemäß einem der Ansprüche 1 bis 13 für die Herstellung eines Medikaments für die Neutralisierung einer antigenen Einheit in einem menschlichen Körperbestandteil.

27. Verwendung gemäß Anspruch 26, wobei die antigene Einheit von einem Organismus stammt, der eine infektiöse Erkrankung auslöst.

28. Verwendung gemäß Anspruch 26, wobei die antigene Einheit ein Zelloberflächenmolekül ist.

29. Verwendung gemäß Anspruch 28, wobei das Zelloberflächemolekül von einem Lymphozyten oder Adipozyten stammt.

30. Verwendung gemäß Anspruch 26, wobei die antigene Einheit ein menschliches Zytokin oder ein menschlichen Chemokin ist.

31. Verwendung gemäß Anspruch 26, wobei die antigene Einheit ein Zelloberflächenmolekül auf einer malignen Krebszelle ist.

32. Verfahren zur Erzeugung eines transgenen nichtmenschlichen Tiers, umfassend mindestens einen Teil der funktionellen menschlichen Immunglobulingene, integriert durch homologe Rekombination ins Genom, wobei der Teil der funktionellen menschlichen Immunglobulingene sich in frame mit Immunglobulingenen, die für das nichtmenschliche Tier endogen sind, umanordnet, um funktionelle Antikörpermoleküle zu codieren, die mindestens einen Teil der menschlichen Immunglobulin konstanten Regionen umfassen und variable Region Aminosäuresequenzen, codiert durch Fragmente von mehr als einem variablen (V)-Regiongen, wobei das transgene nichtmenschliche Tier eine Antikörperdiversität im wesentlichen durch Genkonversion erzeugt und die funktionellen Antikörpermoleküle erzeugt, wenn es immunisiert wird, wobei das Verfahren folgendes umfaßt:
Erzeugung eines ersten mutierten Tiers, umfassend schwere Ketten Immunglobulinloci, wobei konstante und/oder variable Regiongene durch ein oder mehrere Exons des menschlichen schweren Ketten Immunglobulinlocus ersetzt sind, durch genetische Veränderung eines Zellkerns des Tiers, Einführen des Zellkerns in eine erste vom Kern befreite nukleäre Transfereinheitszelle, um eine erste embryonale Stammzelle bereitzustellen, Einführen der ersten nukleären Transfereinheitszelle in einen weiblichen Empfängerwirt, um einen ersten mutierten Neugeborenen zu erzeugen;
Erzeugung eines zweiten mutierten Tiers, umfassend leichte Ketten Immunglobulinloci, wobei konstante und/oder variable Regiongene durch ein oder mehrere Exons des menschlichen leichten Ketten Immunglobulinlocus ersetzt sind, durch genetische Veränderung eines Zellkerns des Tiers, Einführen des Zellkerns in eine zweite vom Kern befreite nukleäre Transfereinheitszelle, um eine zweite embryonale Stammzelle bereitzustellen, Einführen der zweiten nukleären Transfereinheitszelle in einen weiblichen Empfängerwirt, um einen zweiten mutierten Neugeborenen zu erzeugen; und
Züchten von reifen ersten und zweiten mutierten Neugeborenen und Selektion von Tieren, die Antiseren erzeugen können, bestehend im wesentlichen aus Immunglobulinproteinmolekülen, umfassend mindestens einen Teil der menschlichen Immunglobulin konstanten Regionen und variable Region Aminosäuresequenzen, codiert durch Fragmente von mehr als einem variablen (V)-Regiongen, worin (i) die Immunglobulinproteinmoleküle spezifisch an das Immunogen binden, und (ii) wobei das transgene nichtmenschliche Tier Antikörperdiversität im wesentlichen durch Genkonversion erzeugt.

33. Verfahren zur Erzeugung eines transgenen nichtmenschlichen Tiers, das mindestens 1 kg wiegt, umfassend mindestens einen Teil von funktionalen menschlichen Immunglobulingenen, integriert durch homologe Rekombination ins Genom, wobei der Teil der funktionalen menschlichen Immunglobulingene sich in frame mit Immunglobulingenen, die für das nichtmenschliche Tier endogen sind, umanordnet, um funktionelle Antikörpermoleküle zu codieren, die variable Region Aminosäuresequenzen, codiert durch Fragmente von mehr als einem variablen (V)-Regiongen, umfassen, wobei das transgene nichtmenschliche Tier Antikörperdiversität im wesentlichen durch Genkonversion erzeugt und die funktionellen Antikörpermoleküle erzeugt, wenn es immunisiert wird, wobei das Verfahren umfaßt:
Erzeugung eines mutierten Tiers, umfassend schwere und leichte Ketten Immunglobulinloci, worin konstante und/oder variable Regiongene durch ein oder mehrere Exons des menschlichen schweren und/oder Leichtketten Immunblogulinlocus ersetzt sind, durch genetische Veränderung eines Zellkerns des Tiers, Einführen des Zellkerns in eine vom Kern befreite nukleäre Transfereinheitszelle, um eine embryonale Stammstelle bereitzustellen, Einführen der nukleären Transfereinheitszelle in einen weiblichen Empfängerwirt, um einen mutierten Neugeborenen zu erzeugen, und
Züchten von reifen mutierten Neugeborenen und Selektion von Tieren, die in der Lage sind, Antiseren zu erzeugen, bestehend im wesentlichen aus Immunglobulinproteinmolekülen, umfassend mindestens einen Teil der menschlichen schweren Ketten konstanten Region und variable Region Aminosäuresequenzen, codiert durch Fragmente von mehr als einem variablen (V)-Regiongen, worin (i) die Immunglobulinproteinmoleküle spezifisch an das Immunogen binden und (ii) wobei das transgene nichtmenschliche Tier Antikörperdiversität im wesentlichen durch Genkonversion erzeugt.

34. Verfahren gemäß Anspruch 33 oder 34, wobei das Tier zu der Ordnung Lagomorpha gehört.

35. Verfahren gemäß Anspruch 33 oder 34, wobei das Tier ein variables Regiongen proximal zur D-Region umfaßt.

## Revendications

1. Composition d'antisérum polyclonal d'un animal non humain transgénique qui reconnaît spécifiquement un immunogène, dans laquelle ladite composition d'antisérum est essentiellement composée de molécules de protéines immunoglobulines comprenant au moins une partie d'une région constante d'une chaîne lourde humaine et des séquences d'acides aminés d'une région variable codées par des fragments de plus d'un gène d'une région variable (V), dans laquelle (i) lesdites molécules de protéines immunoglobulines se lient spécifiquement audit immunogène, et (ii) ledit animal non humain transgénique génère une diversité d'anticorps essentiellement par conversion génique.

2. Composition d'antisérum polyclonal selon la revendication 1, dans laquelle ledit animal non humain transgénique comprend au moins une partie des gènes fonctionnels d'une chaîne lourde d'immunoglobuline humaine incluant plus d'un gène d'une région V intégré par recombinaison homologue dans son génome.

3. Composition d'antisérum polyclonal selon la revendication 1 ou la revendication 2 composée essentiellement de molécules de protéines immunoglobulines comprenant au moins deux des régions constantes des chaînes lourdes humaines C_{H1}, C_{H2} et C_{H3}.

4. Composition d'antisérum polyclonal selon la revendication 3 composée essentiellement de molécules de protéines immunoglobulines comprenant la région constante de la chaîne lourde humaine C_{H3}.

5. Composition d'antisérum polyclonal selon l'une quelconque des revendications 1 à 4, dans laquelle dans le génome dudit animal non humain, le gène de la région V proximale de la région D est remplacé par un gène d'une région V humaine.

6. Composition d'antisérum polyclonal selon la revendication 5 comprenant des antisérums provenant de divers animaux non humains, dans laquelle le gène de la région V proximale de la région D est remplacé par différents gènes d'une région V humaine.

7. Composition d'antisérum polyclonal selon la revendication 6 comprenant des antisérums provenant de jusqu'à 10 animaux non humains différents.

8. Composition d'antisérum polyclonal selon l'une quelconque des revendications 1 à 7, ayant une affinité d'au moins environ 10⁻⁷.

9. Composition d'antisérum polyclonal selon l'une quelconque des revendications 1 à 7, ayant une affinité d'au moins environ 10⁻⁸.

10. Composition d'antisérum polyclonal selon l'une quelconque des revendications 1 à 7, ayant une affinité d'au moins environ 10⁻⁹.

11. Composition d'antisérum polyclonal selon l'une quelconque des revendications 1 à 10, dans laquelle ledit animal non humain transgénique appartient à l'ordre des *Lagomorpha.*

12. Composition d'antisérum polyclonal selon la revendication 11, dans laquelle ledit animal non humain transgénique est choisi dans le groupe d'animaux constitué par les lapins, les ovins, les bovins, les canins, les félins et les équins.

13. Composition d'antisérum polyclonal selon la revendication 12, dans laquelle ledit animal non humain transgénique est un lapin, un cochon, un mouton ou un bétail.

14. Animal non humain transgénique pesant au moins 1 kg, comprenant au moins une partie des gènes fonctionnels d'une chaîne lourde d'immunoglobuline humaine intégrés par recombinaison homologue dans son génome, dans laquelle ladite partie des gènes fonctionnels d'une chaîne lourde d'immunoglobuline humaine est réarrangée en cadre avec des gènes d'une chaîne lourde d'immunoglobuline endogènes dudit animal non humain pour coder des molécules d'anticorps fonctionnelles qui comprennent au moins une partie d'une région constante d'une chaîne lourde humaine et des séquences d'acides aminés d'une région variable codées par des fragments de plus d'un gène d'une région variable (V), dans laquelle ledit animal non humain transgénique génère une diversité d'anticorps essentiellement par conversion génique et produit lesdites molécules d'anticorps fonctionnelles lorsqu'il est immunisé.

15. Animal non humain transgénique pesant au moins 1 kg, comprenant au moins une partie des gènes fonctionnels d'une chaîne légère d'immunoglobuline humaine intégrés par recombinaison homologue dans son génome, dans laquelle ladite partie des gènes fonctionnels d'une chaîne légère d'immunoglobuline humaine est réarrangée en cadre avec des gènes d'une chaîne légère d'immunoglobuline endogènes audit animal non humain pour coder des molécules d'anticorps fonctionnelles qui comprennent au moins une partie d'une région constante d'une chaîne légère humaine et des séquences d'acides aminés d'une région variable codées par des fragments de plus d'un gène d'une région variable (V), dans laquelle ledit animal non humain transgénique génère une diversité d'anticorps essentiellement par conversion génique et produit lesdites molécules d'anticorps fonctionnelles lorsqu'il est immunisé.

16. Animal non humain transgénique selon la revendication 14 ou la revendication 15, dans laquelle ledit animal non humain transgénique appartient à l'ordre des *Lagomorpha*.

17. Animal non humain transgénique selon la revendication 14, dans laquelle ladite partie des gènes d'une chaîne lourde d'immunoglobuline humaine comprend au moins un gène d'une région constante.

18. Animal non humain transgénique selon la revendication 14, dans laquelle lesdits gènes d'une région variable comprennent le gène d'une région variable proximale de la région D.

19. Animal non humain transgénique selon la revendication 15, dans laquelle ledit gène d'une chaîne légère d'immunoglobuline humaine code une immunoglobuline κ.

20. Procédé *in vitro* pour la neutralisation d'une entité antigénique dans un composant corporel humain, ledit procédé comprenant : mettre en contact ledit composant corporel humain avec une composition d'antisérum selon l'une quelconque des revendications 1 à 13, dans laquelle les molécules de protéines immunoglobulines dans la composition d'antisérum se lient spécifiquement et neutralisent ladite entité antigénique.

21. Procédé selon la revendication 20, dans laquelle ladite entité antigénique provient d'un organisme provoquant une maladie infectieuse.

22. Procédé selon la revendication 20, dans laquelle ladite entité antigénique est une molécule de surface cellulaire.

23. Procédé selon la revendication 22, dans laquelle ladite molécule de surface cellulaire provient d'un lymphocyte ou d'un adipocyte.

24. Procédé selon la revendication 20, dans laquelle ladite entité antigénique est une cytokine humaine ou une chimiokine humaine.

25. Procédé selon la revendication 20, dans laquelle ladite entité antigénique est une molécule de surface cellulaire sur une cellule cancéreuse maligne.

26. Utilisation d'une composition d'antisérum selon l'une quelconque des revendications 1 à 13 pour la préparation d'un médicament destiné à neutraliser une entité antigénique dans un composant corporel humain.

27. Utilisation selon la revendication 26, dans laquelle ladite entité antigénique provient d'un organisme provoquant une maladie infectieuse.

28. Utilisation selon la revendication 26, dans laquelle ladite entité antigénique est une molécule de surface cellulaire.

29. Utilisation selon la revendication 28, dans laquelle ladite molécule de surface cellulaire provient d'un lymphocyte ou d'un adipocyte.

30. Utilisation selon la revendication 26, dans laquelle ladite entité antigénique est une cytokine humaine ou une chimiokine humaine.

31. Utilisation selon la revendication 26, dans laquelle ladite entité antigénique est une molécule de surface cellulaire sur une cellule cancéreuse maligne.

32. Procédé destiné à produire un animal non humain transgénique comprenant au moins une partie des gènes fonctionnels d'immunoglobuline humaine intégrés par recombinaison homologue dans son génome, dans lequel ladite partie des gènes fonctionnels d'immunoglobuline humaine sont réarrangés en cadre avec des gènes d'immunoglobuline endogènes audit animal non humain pour coder des molécules d'anticorps fonctionnelles qui comprennent au moins une partie des régions constantes d'immunoglobuline humaine et des séquences d'acides aminés d'une région variable codées par des fragments de plus d'un gène d'une région variable (V), dans lequel ledit animal non humain transgénique génère une diversité d'anticorps essentiellement par conversion génique et produit lesdites molécules d'anticorps fonctionnelles lorsqu'il est immunisé, ledit procédé comprenant :
la production d'un premier animal muté comprenant des loci d'une chaîne lourde d'immunoglobuline dans lesquels des gènes des régions constantes et/ou variables sont remplacés par un ou plusieurs exons du locus d'une chaîne lourde d'immunoglobuline humaine par modification génétique d'un noyau cellulaire dudit animal, l'introduction dudit noyau cellulaire dans une première unité cellulaire de transfert nucléaire énuclée pour fournir une première cellule souche embryonnaire, l'introduction de ladite première unité cellulaire de transfert nucléaire dans un hôte receveur femelle afin de produire un premier nouveau-né muté ;
la production d'un second animal muté comprenant des loci d'une chaîne légère d'immunoglobuline dans lesquels des gènes des régions constantes et/ou variables sont remplacés par un ou plusieurs exons du locus d'une chaîne légère d'immunoglobuline humaine par modification génétique d'un noyau cellulaire dudit animal, l'introduction dudit noyau cellulaire dans une seconde unité cellulaire de transfert nucléaire énuclée pour fournir une seconde cellule souche embryonnaire, l'introduction de ladite seconde unité cellulaire de transfert nucléaire dans un hôte receveur femelle afin de produire un second nouveau-né muté ;
l'élevage à maturité des premier et second nouveau-nés mutés et la sélection d'animaux capables de produire des antisérum comprenant essentiellement des molécules de protéines immunoglobulines comprenant au moins une partie des régions constantes d'immunoglobuline humaine et des séquences d'acides aminés d'une région variable codées par des fragments de plus d'un gène d'une région variable (V), dans lequel (i) lesdites molécules de protéines immunoglobulines se lient spécifiquement audit immunogène, et (ii) ledit animal non humain transgénique génère une diversité d'anticorps essentiellement par conversion génique.

33. Procédé destiné à produire un animal non humain transgénique pesant au moins 1 kg, comprenant au moins une partie des gènes fonctionnels d'immunoglobuline humaine intégrés par recombinaison homologue dans son génome, dans lequel ladite partie des gènes fonctionnels d'immunoglobuline humaine est réarrangée en cadre avec des gènes d'immunoglobuline endogènes audit animal non humain pour coder des molécules d'anticorps fonctionnelles qui comprennent des séquences d'acides aminés d'une région variable codées par des fragments de plus d'un gène d'une région variable (V), dans lequel ledit animal non humain transgénique génère une diversité d'anticorps essentiellement par conversion génique et produit lesdites molécules d'anticorps fonctionnelles lorsqu'il est immunisé, ledit procédé comprenant :
la production d'un animal muté comprenant des loci des chaînes lourdes et légères d'immunoglobuline dans lesquels des gènes des régions constantes et/ou variables sont remplacés par un ou plusieurs exons du locus des chaînes lourdes et/ou légères d'immunoglobuline humaine par modification génétique d'un noyau cellulaire dudit animal, l'introduction dudit noyau cellulaire dans une unité cellulaire de transfert nucléaire énuclée pour fournir une cellule souche embryonnaire, l'introduction de ladite unité cellulaire de transfert nucléaire dans un hôte receveur femelle afin de produire un nouveau-né muté ; et
l'élevage à maturité des nouveau-nés mutés et la sélection d'animaux capables de produire des antisérum comprenant essentiellement des molécules de protéines immunoglobulines comprenant au moins une partie d'une région constante d'une chaîne lourde humaine et des séquences d'acides aminés d'une région variable codées par des fragments de plus d'un gène d'une région variable (V), dans lequel (i) lesdites molécules de protéines immunoglobulines se lient spécifiquement audit immunogène, et (ii) ledit animal non humain transgénique génère une diversité d'anticorps essentiellement par conversion génique.

34. Procédé selon la revendication 33 ou la revendication 34, dans lequel ledit animal appartient à l'ordre des *Lagomorpha*.

35. Procédé selon la revendication 33 ou la revendication 34, dans lequel ledit animal comprend un gène d'une région variable proximale de la région D.
